# EUROPEAN PATENT APPLICATION

(11) **EP 4 083 019 A1**
(43) Date of publication of application: **02.11.2022**
(21) Application number: 20908276.7
(22) Date of filing: 24.12.2020
(51) Int. Cl.: C07C 331/20, A61P 35/00

(54) **MAGNOLOL AND SULFORAPHANE CONJUGATE, AND PREPARATION METHOD THEREFOR**

(30) Priority: 26.12.2019 CN 201911370139
(71) Applicant: Shenzhen Zhenxing Biomedicine Research Center Co., Ltd., Shenzhen, Guangdong 518063 (CN)
(72) Inventor: WU, Zhengzhi, Shenzhen, Guangdong 518028 (CN); TAO, Cheng, Shenzhen, Guangdong 518028 (CN); CHEN, Jian, Shenzhen, Guangdong 518028 (CN); ZENG, Yongchang, Shenzhen, Guangdong 518028 (CN); LIU, Jieren, Shenzhen, Guangdong 518028 (CN); LI, Limin, Shenzhen, Guangdong 518028 (CN)
(74) Representative: Uexküll & Stolberg
(86) International application number: PCT/CN2020/139058
(87) International publication number: WO 2021/129747

(57) **Abstract**

Disclosed in the present invention are a magnolol and sulforaphane conjugate, and a preparation method and use thereof. The magnolol and sulforaphane conjugate comprises C₂₅H₂₉NO₂S₂ **(CT-1),** C₂₅H₂₉NO₄S₂ **(CT-2),** and C₂₅H₂₉NO₃S₂ (**CT-3**). By using the drug combination principle, the present invention achieved the first synthesis of the magnolol and sulforaphane conjugate **(CT-1, CT-2, CT-3**). According to biological evaluation, the conjugate has an antitumor effect remarkably superior to that of magnolol and sulforaphane, has a broad spectrum of antitumor activity and strong druggability. It is an antitumor compound with development potential, and also provides new ideas and approaches for development of a novel antitumor drug.

## Description

### FIELD OF THE INVENTION

The present invention relates to the field of medicines, and more particularly relates to a magnolol and sulforaphane conjugate, and method of preparation and use thereof.

### BACKGROND OF THE INVENTION

Malignant tumors have become the second leading cause of human death and one of the major diseases that threaten human health. China is rich in natural medicinal resources, and it is of great significance to screen active monomers from traditional Chinese medicines and utilize medicinal chemistry methods for structural modification and alternation for the discovery of new anti-tumor drugs.

Magnolol ( 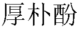 , also known as 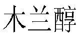 ) is one of the main components of the traditional Chinese medicine magnolia officinalis, with moderate antitumor activity (Lin, S.Y; Liu, J.D.; Chang, H.C. Magnolol Suppresses Proliferation of Cultured Human Colon and Liver Cancer Cells by Inhibiting DNA Synthesis and Activating Apotosis [J]. J. Cell Biochem., 2002, 84, 532-544). In recent years, researches on the anti-tumor mechanism of magnolol have become a hot spot in the field of medicines, and medicinal chemists have further enhanced anti-tumor activities by structurally modifying magnolol (Tang, H.; Zhang, Y; Li, D. Discovery and Synthesis of Novel Magnolol Derivatives with Potent Anticancer Activity in Non-Small Cell Lung Cancer [J]. Eur. J. Med. Chem., 2018, 156, 190-205).

Sulforaphane mainly exists in cruciferous plants, and is the isothiocyanate compound with the strongest anti-cancer ability in nature (Springer T.A. Traffic Signals for Lymphocyte Recirculation and Leukocyte Emigration: the Multistep Paradigm [J]. Cell, 1994, 76, 301-314). Both magnolol and sulforaphane have good medicinal value. Therefore, it is of great theoretical significance and practical value to use these two types of compounds as lead compounds for structural modification and transformation to develop new anti-tumor drugs.

### SUMMARY OF THE INVENTION

In order to solve the problems of the prior technologies, the present invention provided a magnolol and sulforaphane conjugate, and method of preparation and use thereof.

In a first aspect, the present invention provided a magnolol and sulforaphane conjugate (or splicing compound), which has a structure of: 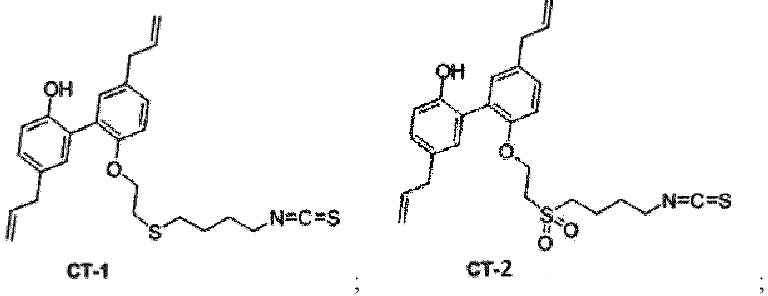 or

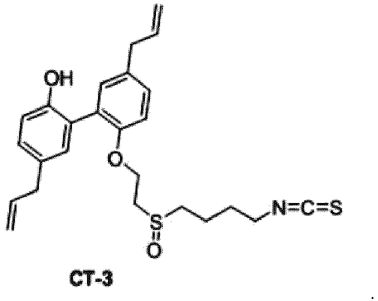

In a second aspect, the present invention provided a method of preparation of magnolol and sulforaphane conjugate, the method comprising:
dissolving magnolol in tetrahydrofuran, adding sodium hydride and 1,2-dibromoethane, running reaction at 60° C for 3 hours, and then adding water, the resulting mixture being extracted, organic phase being dried and concentrated to give compound **2**;
dissolving compound **2** in N,N-dimethylformamide, adding sodium sulfide, running reaction at 25°C for 2 hours, adjusting pH to 1, and resulting mixture being allowed to stand and then filtered, precipitate being collected and dissolved in N,N-dimethylformamide, adding potassium carbonate and 4-bromo-1-butylamine, running reaction at 25°C for 12 hours, reaction solution being concentrated and dissolved in tetrahydrofuran, adding triethylamine and carbon disulfide at 0°C, running reaction at 25°C for 2 hours, adding water, extracted, organic phase being dried and concentrated to give compound **CT-1**.

The method may further comprise:
dissolving compound **CT-1** in methylene chloride, adding m-chloroperoxybenzoic acid, running reaction at 25° C for 1 hour, then adding a saturated aqueous solution of sodium bicarbonate, resulting mixture being filtered and extracted, and organic phase being dried and concentrated to give compound **CT-2** and compound **CT-3**.

In a third aspect, the present invention provided a use of magnolol and sulforaphane conjugate, wherein the magnolol and sulforaphane conjugate is used in a therapeutically effective amount for manufacuture of a medicament for the treatment of cancer.

The technologies of the present invention have some advantages, including: the magnolol and sulforaphane conjugates, or splicing compounds (CT-1, CT-2, CT-3) are synthesized for the first time via the approach of drug splicing. According to biological evaluation, the anti-tumor effect of the magnolol and sulforaphane conjugates is significantly higher than that of magnolol and sulforaphane, respectively. The magnolol and sulforaphane conjugates have broad-spectrum anti-tumor activity and strong druggability, and the conjugates have great potential for the development as novel anti-tumor drugs.

### DETAILED DESCRIPTION OF THE EMBODIMENTS

In order to make the objectives, advantages and various aspects of the invention be best understood, the embodiments of the present invention will be further described in detail below with reference to the accompanying drawings.

### EXAMPLES

### I. Preparation of magnolol and sulforaphane conjugates

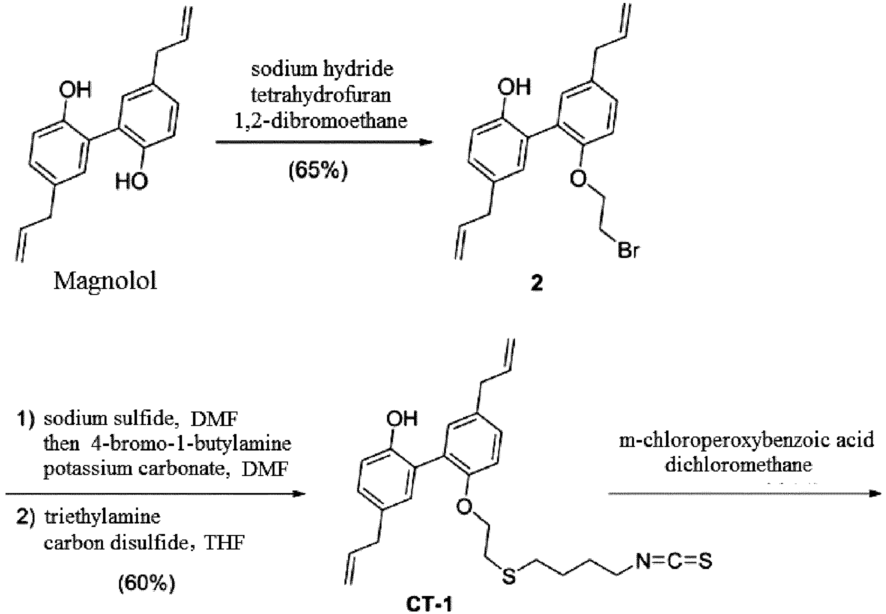

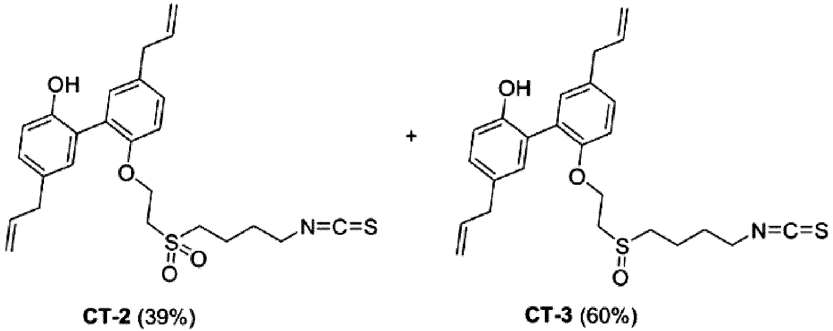

(1) Magnolol (2.66 g, 10 mmol) was dissolved in tetrahydrofuran (100 mL), and 60% sodium hydride (40 mmol) and 1,2-dibromoethane (40 mmol) were added, the reaction was run at 60°C for 3 hours, then water (30 mL) was added, and the resulting material was extracted with ethyl acetate (100 mL), the organic phase was dried with sodium sulfate, concentrated, and separated with a silica gel column to give Compound **2** (2.4 g, 6.5 mmol) as a pale yellow liquid in a yield of 65%.
   Compound **2:** R_{f} = 0.8 (petroleum ether/EtOAc, 10:1); ¹H NMR (400 MHz, Chloroform-*d*) δ 7.14 (dd, *J* = 6.5, 2.2 Hz, 2H), 7.11-7.02 (m, 2H), 6.94 (dd, *J* = 8.6, 4.3 Hz, 2H), 6.07 (s, 1H), 6.04-5.87 (m, 2H), 5.12-5.02 (m, 4H), 4.22 (t, *J* = 6.2 Hz, 2H), 3.45 (t, *J* = 6.2 Hz, 2H), 3.39-3.30 (m,4H); ¹³C NMR (100 MHz, CDCl₃) δ 152.6, 151.6, 137.7, 137.1, 134.7, 132.5, 132.2, 131.1, 129.3, 129.1, 127.9, 125.8, 117.4, 115.9, 115.4, 114.2, 114.2, 69.6, 39.3, 39.2, 28.6; HRMS (ESI): *m*/*z* calcd for C₂₀H_{2I}BrNaO₂ [M+Na]⁺: 395.0623, found: 395.0706.
(2) Compound **2** (3.72 g, 10 mmol) was dissolved in N,N-dimethylformamide, (100 mL), sodium sulfide (25 mol) was added, the reaction was run at 25°C for 2 hours, then hydrochloric acid (2 mol/L) was added to adjust pH to 1, and the resulting material was allowed to stand, filtered; the precipitate was collected and dissolved in N,N-dimethylformamide (50 mL), potassium carbonate (25 mol) and 4-bromo-1-butylamine 25 mmol) were added, and the reaction was run at 25°C for 12 hours; the resulting solution was concentrated and dissolved in tetrahydrofuran (30 mL), then triethylamine (15 mol) and carbon disulfide (5 mol) were added at 0 °C, and the reaction was run at 25 °C for 2 hours, water (30 mL) was added, the resulting material was extracted with ethyl acetate (50 mL), and the organic phase was dried with sodium sulfate, concentrated, and separated with a silica gel column to give Compound **CT-1** (2.6 g, 6 mmol) as a pale yellow liquid in a yield of 60%.
   Compound **CT-1:** R_{f} = 0.2 (petroleum ether/EtOAc, 5:1); ¹H NMR (400 MHz, Chloroform-*d*) δ 7.24-7.03 (m, 4H), 6.98 (dd*, J* = 8.2, 4.7 Hz, 2H), 6.01 (dddd, *J* = 14.6, 10.2, 7.0, 3.3 Hz, 2H), 5.21-4.99 (m, 4H), 4.20 (t, *J* = 6.3 Hz, 2H), 3.49 (t, *J* = 6.3 Hz, 2H), 3.41 (t, *J* = 6.1 Hz, 4H), 2.83 (t, *J* = 6.3 Hz, 2H), 2.48 (t, *J* = 6.9 Hz, 2H), 1.75-1.66 (m, 2H), 1.66-1.56 (m, 2H); ¹³C NMR (100 MHz, CDCl₃) δ 153.0, 151.9, 137.8, 137.3, 134.3, 132.6, 132.4, 131.2, 129.3, 129.2, 127.6, 126.2, 117.4, 115.9, 115.5, 113.4, 69.7, 44.6, 39.4, 39.3, 31.7, 30.9, 29.6, 28.7, 26.3; HRMS(ES1): *m*/*z* calcd for C₂₅H₂₉NNaO₂S₂ [M+Na]⁺: 462.1537, found:462.1770.
(3) Compound **CT-1** (4.39 g, 10 mmol) was dissolved in dichloromethane (100 mL) at 25 °C (room temperature), m-chloroperoxybenzoic acid (11 mol) was added, the reaction was run at 25 °C for 1 hour, and a saturated aqueous solution of sodium bicarbonate (30 mL) was added, the resulting material was extracted with dichloromethane (50 mL), the organic phase was dried with sodium sulfate, concentrated, and separated with a silica gel column to give Compound **CT-2** (1.8 g, 3.9 mmol) as a pale yellow liquid in a yield of 39% and Compound **CT-3** (2.7g, 6 mmol) as a pale yellow liquid in a yield of 60%.

Compound **CT-2**: R_{f} = 0.6 (petroleum ether/EtOAc, 1:1): ¹H NMR (400 MHz, Chloroform-d) δ 7.22 (dd, *J* = 8.4, 2.3 Hz, 1H), 7.13 (dd, *J* = 8.0, 2.2 Hz, 2H), 7.01 (d, *J* = 2.2 Hz, 1H), 6.93 (dd, *J* = 16.4, 8.3 Hz, 2H), 6.02-5.88 (m, 2H), 5.16-5.01 (m, 4H), 4.42 (t, *J* = 5.1 Hz, 2H), 3.45-3.34 (m, 6H), 3.30 (t, *J* = 5.2 Hz, 2H), 2.66 (t, *J* = 7.2 Hz, 2H), 1.72-1.65 (m, 2H), 1.60-1.52 (m, 2H); ¹³C NMR (100 MHz, CDCI₃) δ 152.9, 151.5, 137.5, 137.1, 134.6, 132.5, 132.4, 131.1, 129.7, 129.2, 126.1, 125.2, 116.1, 115.8, 112.5, 63.0, 53.5, 53.3, 44.5, 39.3, 39.2, 29.7, 28.4, 18.7; HRMS (ESI): *m*/*z* calcd for C₂₅H₂₉NNaO₄S₂ [M+Na]⁺: 494.1436, found : 494.1854.

Compound **CT-3**: R_{f} = 0.2 (petroleum ether/EtOAc, 1:1); ¹H NMR (400 MHz) Chloroform-d) δ 7.20 (dd, *J* = 8.4, 2.3 Hz, 1H), 7.16-7.07 (m, 2H), 7.06-6.96 (m, 2H), 6.91 (d*, J* = 8.2 Hz, 1H), 6.04-5.92 (m, 2H), 5.17-5.04 (m, 4H), 4.55-4.32 (m, 2H), 3.49 (t, *J* = 6.2 Hz, 2H), 3.44-3.31 (m, 4H), 3.13 (ddd, *J* = 13.0, 8.5, 4.2 Hz, 1H), 2.95 (ddd, *J* = 13.9, 5.5, 3.4 Hz, 1H), 2.71-2.57 (m, 1H), 2.49 (dt, *J* = 13.3, 7.5 Hz, 1H), 1.81-1.62 (m, 4H); ¹³C NMR (101 MHz, CDCl₃) δ 153.1, 151.8, 137.7, 137.2, 134.5, 132.3, 132.1, 131.1, 129.3, 129.2, 127.4, 125.8, 116.7, 116.0, 115.6, 113.4, 61.7, 51.6, 51.4, 44.5, 39.4, 39.3, 28.8, 20.2; HRMS (ESI): *m*/*z* calcd for C₂₅H₂₉NNaO₃S₂ [M+Na]⁺: 478.1487, found: 478.2287.

### II. Effects of magnolol and sulforaphane conjugates on the proliferation of human lung cancer cell line NCI-H460

### 1. Experimental materials

### 1.1 Cell lines

Human lung cancer cell line NCI-H460.

### 1.2 Medicinal materials, reagents and instruments

Fetal Bovine Serum (Hyclone), RPMI-1640 Medium (Gibco), DMSO (Sigma), CCK8 Kit (Dalian Meilun Biotech), Trypsin (Gibco), SpectraMax 190 Microplate Reader (Molecular Devices), Low-Speed Centrifuge (Anhui Zhongke Zhongjia Scientific Instrument), Ultra-Clean Bench (Medical Equipment Factory of Shanghai Boxun Industrial Co., Ltd.), and Inverted Microscope (Mike Audi Co., Ltd.).

### 2. Experimental method

2.1 Cultivation of NCI-H460 cells. The cells, when grow to the logarithmic growth phase, were digested with 0.25% trypsin, centrifuged at 1000×g for 5 min, and the cell pellet were re-suspended with 1640 medium containing 10% FBS and inoculated in a 96-well culture plate at a density of 5000 cells/well, and then cultured overnight at 37°C, 5% CO₂ and saturated humidity.

2.2 Six duplicate wells were set in each group, and drugs were added to each well according to the following conditions, and then cultured for 24 h.

Control group: The group without drugs.

Magnolol group: Magnolol added at a final concentration of 20 µM.

CT-1 group: CT-1 added at a final concentration of 20 µM.

CT-2 group: CT-2 added at a final concentration of 20 µM.

CT-3 group: CT-3 added at a final concentration of 20 µM.

The above cells were intervened for 24 hours, and then the effects of drugs on tumor cell proliferation were detected by using CCK8 kit. The specific operations are as follows: 10 µL of CCK8 solution was added to each well and cultured for 2 h, and then the absorbance (OD) value at a wavelength of 450 nm was measured on a microplate reader, and the cell survival rate of the other groups was calculated on the basis of the cell survival rate of the control group as 100%.

### 3. Experimental results

**Table 1. Effects of the magnolol and sulforaphane conjugates on proliferation of human lung cancer cell line NCI-H460**

| Groups | Inhibition Rate (%) |
|---|---|
| Control group | 0.00 |
| Magnolol group | 1.20 |
| CT-1 group | 18.41 |
| CT-2 group | 70.34 |
| CT-3 group | 98.99 |

As shown in Table 1, the results indicate that the magnolol and sulforaphane conjugates CT-1, CT-2 and CT-3 can significantly inhibit the proliferation of human lung cancer cells NCI-H460, and the inhibitory effect is significantly higher than that of the positive control drug magnolol, while CT-3 has the strongest antitumor activity.

### III. Effect of CT-3 on proliferation of seven human cancer cell lines

### 1. Experimental materials

### 1.1 Cell lines

Human glioma cell line U251, human lung cancer cell line H1650, human liver cancer cell line HepG2, human breast cancer cell line MDA-MB-231, human gastric cancer cell line AGS, human colon cancer cell line LOVO, human ovarian cancer cell line OVCAR3.

### 1.2 Medicinal materials, reagents and instruments

Fetal Bovine Serum (Hyclone), RPMI-1640 Medium (Gibco), DMSO (sigma), CCK8 Kit (Dalian Meilun Biotech), Trypsin (Gibco), SpectraMax 190 Microplate Reader (Molecular Devices), Low-Speed Centrifuge (Anhui Zhongke Zhongjia Scientific Instrument), Ultra-Clean Bench (Medical Equipment Factory of Shanghai Boxun Industrial Co., Ltd.), and Inverted Microscope (Mike Audi Co., Ltd.).

### 2. Experimental method

2.1 Cultivation of the above 7 kinds of cancer cells. The cells, when grow to the logarithmic growth phase, were digested with 0.25% trypsin, centrifuged at 1000×g for 5 min, and the cell pellet were re-suspended with 1640 medium containing 10% FBS and inoculated in a 96-well culture plate at a density of 5000 cells/well, and then cultured overnight at 37°C, 5% CO₂ and saturated humidity.

2.2 Each group was set up with 6 duplicate wells, and drugs were added to each well according to the following conditions, and then cultured for 24 h.

Control group: The group without drugs.

Positive control groups: Magnolol group and sulforaphane group respectively divided into 3 groups:
Positive control group 1: Magnolol added at a final concentration of 5.0 µM;
Positive control group 2: Magnolol added at a final concentration of 10.0 µM;
Positive control group 3: Magnolol added at a final concentration of 20.0 µM;
Positive control group 4: Sulforaphane added at a final concentration of 5.0 µM;
Positive control group 5: Sulforaphane added at a final concentration of 10.0 µM;
Positive control group 6: Sulforaphane added at a final concentration of 20.0 µM.

CT-3 groups: Divided into 3 groups:
CT-3 group 1: CT-3 added at a final concentration of 5.0 µM;
CT-3 group 2: CT-3 added at a final concentration of 10.0 µM;
CT-3 group 3: CT-3 added at a final concentration of 20.0 µM.

The above cells were intervened for 24 hours, and then the effects of drugs on tumor cell proliferation were detected by using CCK8 kit. The specific operations are as follows: 10 µL of CCK8 solution was added to each well and cultured for 2 h, and then the absorbance (OD) value at a wavelength of 450 nm was measured on a microplate reader, and the cell survival rate of the other groups was calculated on the basis of the cell survival rate of the control group as 100%.

### 3. Experimental results

**Table 2. Half maximal inhibitory concentration (IC50) (µM) of CT-3 against seven human cancer cell lines**

| Cell Lines | CT-3 | Magnolol | Sulforaphane |
|---|---|---|---|
| U251 | 7.75 | > 45 | 22.47 |
| H1650 | 6.92 | > 45 | 22.01 |
| HepG2 | 6.24 | > 45 | 20.99 |
| MDA-MB-231 | 7.48 | > 45 | 22.18 |
| AGS | 8.02 | > 45 | 18.14 |
| LOVO | 5.10 | > 45 | 18.52 |
| OVCAR3 | 5.34 | > 45 | 18.44 |

As shown in Table 2, the results indicate that CT-3 can significantly inhibit the proliferation of 7 kinds of human cancer cells, and its IC₅₀ is between 5.10 and 8.02 µM, which is significantly lower than that of the positive control drugs of magnolol (> 45 µM) and sulforaphane (18.41 - 22.47 µM); which indicated that the antitumor activity of CT-3 is significantly higher than that of the positive control drugs magnolol and sulforaphane.

### IV. Effect of CT-3 on apoptosis of human lung cancer cell line NCI-H460

### 1. Experimental materials

### 1.1 Cell lines

Human lung cancer cell line NCI-H460.

### 1.2 Medicinal materials, reagents and instruments

Fetal Bovine Serum (Hyclone), RPMI-1640 Medium (Gibco), DMSO (sigma), CCK8 Kit (Dalian Meilun Biotech), Trypsin (Gibco), SpectraMax 190 Microplate Reader (Molecular Devices), Low-Speed Centrifuge (Anhui Zhongke Zhongjia Scientific Instrument), Ultra-Clean Bench (Medical Equipment Factory of Shanghai Boxun Industrial Co., Ltd.), and Inverted Microscope (Mike Audi Co., Ltd.), Apoptosis Detection Kit and Flow Cytometry (BD).

### 2. Experimental method

2.1 Cultivation of NCI-H460 cells. The cells, when grow to the logarithmic growth phase, were digested with 0.25% trypsin, centrifuged at 1000×g for 5 min, and the cell pellet were re-suspended with 1640 medium containing 10% FBS and inoculated in a 96-well culture plate at a density of 3.0 × 10⁵ cells/well, and then cultured overnight at 37°C, 5% CO₂ and saturated humidity.

2.2 Each group was set up with 3 duplicate wells, and drugs were added to each well according to the following conditions, and then cultured for 24 h.

Control group: The group without drugs.

Magnolol group: Magnolol added at a final concentration of 10.0 µM.

Sulforaphane group: Sulforaphane added at a final concentration of 10.0 µM.

CT-3 group: CT-3 added at a final concentration of 10.0 µM.

The above cells were intervened for 24 hours, and then the effect of CT-3 on tumor cell apoptosis was determined by apoptosis detection kit and flow cytometry.

### 3. Experimental results

**Table 3. Effects of CT-3 on apoptosis of human lung cancer cell line NCI-H460**

| Groups | Apoptosis Rate (%) |
|---|---|
| Control group | 2.31 |
| Magnolol group | 3.12 |
| Sulforaphane group | 18.90 |
| CT-3 group | 73.82 |

As shown in Table 3, the results indicate that CT-3 can significantly induce the apoptosis of human lung cancer cells NCI-H460, and the effect is significantly better than the positive control drugs magnolol and sulforaphane.

### V. Effect of CT-3 on proliferation of human colon cancer cell line SW480

### 1. Experimental materials

### 1.1 Cell lines

Human colon cancer cell line SW480.

### 1.2 Medicinal materials, reagents and instruments

Fetal Bovine Serum (Hyclone), RPMI-1640 Medium (Gibco), DMSO (sigma), CCK8 Kit (Dalian Meilun Biotech), Trypsin (Gibco), SpectraMax 190 Microplate Reader (Molecular Devices), Low-Speed Centrifuge (Anhui Zhongke Zhongjia Scientific Instrument), Ultra-Clean Bench (Medical Equipment Factory of Shanghai Boxun Industrial Co., Ltd.), and Inverted Microscope (Mike Audi Co., Ltd.), Apoptosis Detection Kit and Flow Cytometry (BD).

### 2. Experimental method

2.1 Cultivation of SW480 cells. The cells, when grow to the logarithmic growth phase, were digested with 0.25% trypsin, centrifuged at 1000×g for 5 min, and the cell pellet were re-suspended with 1640 medium containing 10% FBS and inoculated in a 6-well culture plate at a density of 3.0×10⁵ cells/well, and then cultured overnight at 37°C, 5% CO₂ and saturated humidity.

2.2 Each group was set up with 3 duplicate wells, and drugs were added to each well according to the following conditions, and then cultured for 24 h.

Control group: The group without drugs.

Magnolol group: Magnolol added at a final concentration of 10.0 µM.

Sulforaphane group: Sulforaphane added at a final concentration of 10.0 µM.

CT-3 group: CT-3 was added at a final concentration of 10.0 µM.

The above cells were intervened for 24 hours, and then the effect of CT-3 on tumor cell apoptosis was determined by apoptosis detection kit and flow cytometry.

### 3. Experimental results

**Table 4. Effect of CT-3 on apoptosis of human colon cancer cell line SW480**

| Groups | Apoptosis Rate (%) |
|---|---|
| Control group | 3.11 |
| Magnolol group | 4.02 |
| Sulforaphane group | 19.99 |
| CT-3 group | 61.87 |

As shown in Table 4, the results indicate that CT-3 can significantly induce the apoptosis of human colon cancer cell line SW480, and the effect is significantly better than the positive control drugs magnolol and sulforaphane.

### VI. Effect of CT-3 on apoptosis of human hepatic normal cell line LO2

### 1. Experimental materials

### 1.1 Cell lines

Human liver normal cell line LO2.

### 1.2 Medicinal materials, reagents and instruments

Fetal Bovine Serum (Hyclone), RPMI-1640 Medium (Gibco), DMSO (sigma), CCK8 Kit (Dalian Meilun Biotech), Trypsin (Gibco), SpectraMax 190 Microplate Reader (Molecular Devices), Low-Speed Centrifuge (Anhui Zhongke Zhongjia Scientific Instrument), Ultra-Clean Bench (Medical Equipment Factory of Shanghai Boxun Industrial Co., Ltd.), and Inverted Microscope (Mike Audi Co., Ltd.), Apoptosis Detection Kit and Flow Cytometry (BD).

### 2. Experimental method

2.1 Cultivation of the above cancer cells. The cells, when grow to the logarithmic growth phase, were digested with 0.25% trypsin, centrifuged at 1000×g for 5 min, and the cell pellet were re-suspended with 1640 medium containing 10% FBS and inoculated in a 6-well culture plate at a density of 3.0×10⁵ cells/well, and then cultured overnight at 37°C, 5% CO₂ and saturated humidity.

2.2 Each group was set up with 3 duplicate wells, and drugs were added to each well according to the following conditions, and then cultured for 24 h.

Control group: The group without drugs.

Magnolol group: Magnolol added at a final concentration of 10.0 µM.

Sulforaphane group: Sulforaphane added at a final concentration of 10.0 µM.

CT-3 group: CT-3 added at a final concentration of 10.0 µM.

The above cells were intervened for 24 hours, and then the effect of CT-3 on tumor cell apoptosis was determined by apoptosis detection kit and flow cytometry.

### 3. Experimental results

**Table 5. Effect of CT-3 on apoptosis of human hepatic normal cell line LO2**

| Groups | Apoptosis Rate (%) |
|---|---|
| Control group | 2.16 |
| Magnolol group | 4.71 |
| Sulforaphane group | 6.52 |
| CT-3 group | 6.48 |

As shown in Table 5, the results indicate that CT-3 has no obvious inducing effect on the apoptosis of human liver normal cell line LO2, which indicates that CT-3 has no obvious toxic and side effects on normal cells.

Conclusion: CT-3 can inhibit the proliferation of various tumor cells with its anti-tumor activity significantly superior to that of the positive control drugs magnolol and sulforaphane, and no obvious toxic and side effects on normal cells. It is an anti-tumor compound with potential for development, and can be directly used for the treatment of diseases and the preparation of medications.

The above-mentioned serial numbers of the embodiments of the present invention are only for description, and do not represent the order of preference of the embodiments.

Although particular embodiments and examples have been described herein in detail, the above description has been done by way of example for purposes of illustration only, and is not intended to be limiting with respect to the scope of the invention. In particular, it is contemplated by the inventor that various substitutions, alterations, and modifications may be made to the invention without departing from the scope of the invention as claimed.

## Claims

1. A magnolol and sulforaphane conjugate, which has a structure of: 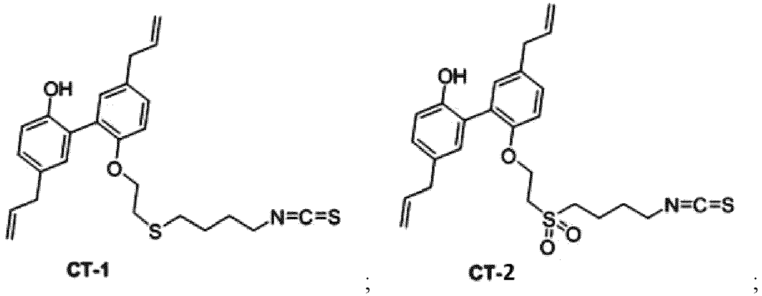 or

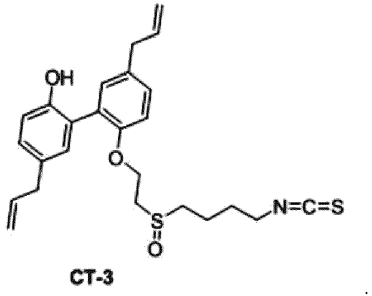

2. A method of preparation of magnolol and sulforaphane conjugate, the method comprising:
dissolving magnolol in tetrahydrofuran, adding sodium hydride and 1,2-dibromoethane, running reaction at 60° C for 3 hours, and then adding water, resulting mixture being extracted, organic phase being dried and concentrated to give compound 2;
dissolving compound **2** in N,N-dimethylformamide, adding sodium sulfide, running reaction at 25°C for 2 hours, adjusting pH to 1, and resulting mixture being allowed to stand and then filtered, precipitate being collected and dissolved in N,N-dimethylformamide, adding potassium carbonate and 4-bromo-1-butylamine, running reaction at 25°C for 12 hours, reaction solution being concentrated and dissolved in tetrahydrofuran, adding triethylamine and carbon disulfide at 0°C, running reaction at 25°C for 2 hours, adding water, extracted, organic phase being dried and concentrated to give compound **CT-1**.

3. The method according to claim 2, further comprising:
dissolving compound **CT-1** in methylene chloride, adding m-chloroperoxybenzoic acid, running reaction at 25° C for 1 hour, then adding a saturated aqueous solution of sodium bicarbonate, resulting mixture being filtered and extracted, and organic phase being dried and concentrated to give compound **CT-2** and compound **CT-3**.

4. A method of use of the magnolol and sulforaphane conjugate of claim 1, wherein the magnolol and sulforaphane conjugate is used for preparation of a medicament for treatment of cancer.
